# EUROPEAN PATENT APPLICATION

(11) **EP 1 407 771 A1**
(43) Date of publication of application: **14.04.2004**
(21) Application number: 02754130.9
(22) Date of filing: 14.06.2002
(51) Int. Cl.: A61K 31/4375, C07G 5/00, A61P 29/00, A61P 35/00

(54) **SOPHORIDINE AND USE THEREOF AS AN ANALGESIC**

(30) Priority: 14.06.2001 CN 01114066
(71) Applicant: Tonghua Fangda Pharmaceuticals, Ltd, Tonghua, Jilin 134000 (CN); Ju, Hongfu, Tonghua, Jilin (CN)
(72) Inventor: JU, Hongfu, Jilin 134000 (CN)
(74) Representative: Bernasconi, Jean Raymond
(86) International application number: PCT/CN2002/000421
(87) International publication number: WO 2002/102379

(57) **Abstract**

The present invention relates to use of natural or chemically synthetic sophoridine and its analogues or derivatives as an analgesic. The present invention further related to pharmaceutical compositions comprising alkaloid compounds having diquinolizidine-like structure selected from the group consisting of sophoridine, sophocarpine, matrine, oxymatrine, malanine, aloperine, sophoramine, and pharmaceutically acceptable salts thereof, and one or more pharmacentically acceptable carriers or excipients. The pharmaceutical compositions of the present invention can be. used for eliminating or alleviating acute or chronic pain without suffering from the undesirable tolerance and addiction with continued usage.

## Description

### FIELD OF THE INVENTION

The present invention generally relates to the use of alkaloids derived from the leguminous Sophora alopecuroides plant as an analgesic, in particularly; relates to the use of natural or chemically synthetic sophoridine and analogues or derivatives thereof as an analgesic.

### BACKGROUND OF THE INVENTION

Generally, the existing analgesics can be divided into two categories: one is narcotic analgesics, which excite receptors of the central nerve system to alleviate severe or sharp pain caused by, for example, trauma, operation, disease and the like. Narcotic analgesics include an narcotic agonists comprising the morphine group, the pethidine group and the methadone group, and narcotic antagonists ( agonist-antagonists ) comprising the morphine-like or nalorphine-like analgesic , such as morphine, heroin, codeine, oxymorphine and levallorphan. Almost all of narcotic analgesics suffer from the adverse effects of tolerance and addiction with repeated use, and possible respiratory and circulatory inhibition. The potential for the development of tolerance and physical dependence with repeated opioid use is a characteristic feature of all the opioid drugs, and the possibility of developing psychological dependence (i.e., addiction) is one of the major concerns in the use of the treatment of pain with opioids. The another class of analgesic is non-steroidal anti-inflammatory drugs ( NSAIDS_{S} ), which inhibits the production of prostaglandin from arachidunic acid thereby widely administered in treatment of mild to severe pain or treatment of inflammatory states, and including aspirin, iburophen, indornethacin, acetaminophen and the like. Nevertheless, these drugs often leads to adverse side-effects, for example, ulcer and bleeding of upper digestive tract.

Sophoridine is an alkaloid first extracted and purified by Oreknov (A. Oreknov, *C.A*., 27,4234) from the leguminous plant Sophora alopecuroides. Until now, it is have been extracted more than 10 alkaloids that have diquinolizidine-like structure from Sophora alopecuroides plant, comprising Matrine, Aloperine, Malanine, Sophocarpine, Oxymatrine, Sophoramine, and Sophoridine. Like many of plant alkaloids from natural source, alkaloids extracted from Sophora alopecuroides have variety of biological activities. For example, matrine has been used extensively as a systemic anti-inflammatory agent for a very long period of time in ancient China (Cho *et al*., IRCS Med. Sci. 14:441-2, 1986). Xuemei Li et al. (Xuemci Li *et al*., Chinese Journal of Pharmacology 8(2):153-158,1987 and China Patent No. 93100881.6) described anti-cancer activities of Sophoridinc derived from Sophora alopecuroides. Further, US Patent No. 5,041,450 disclosed a use of matrine and derivatives thereof in reducing or preventing ocular inflammation. Also, US Patent No.5,908,628 disclosed a pharmaceutical composition consists of twelve natural occurring Chinese traditional herbs comprising Sophora with anti-pain and anti-inflammatory activities. However, there are no report on Sophoridine and analogues or derivatives thereof isolated from the natural sources or chemical synthesized in particularly used for the patients with advanced cancers as an analgesic or as an agent for both analgesic and anti-tumor.

### OBJECTS OF THE INVENTION

Therefore, the first object of the present invention is provide a pharmaceutical compositions useful for treatment or alleviation of pain caused by various reasons in mammals including human, comprising a therapeutically effective amount of diquinolizidine-like compounds as a essentially active ingredient, and one or more pharmaceutically acceptable carriers or excipients.

According to another preferred embodiment of the present invention, wherein said diquinoliziduie-like compounds are obtained from natural or chemical synthetic sources.

In a more preferred embodiment of the present invention, wherein diquinolizidine-like compounds arc selected from the group consisting of matrine, aloperine, malanine, sophocarpine, oxymatrine, sophoramine and Sophoridine, and pharmaceutically acceptable salts thereof.

According to a particular preferred embodiment of the present invention, wherein said diquinolizidine-like compounds are selected from sophoridine and analogues or derivatives thereof.

According to another particular preffered embodiment of the present invention, wherein sophoridine and analogues or derivatives thereof are derived from leguminous Sophora alopecuroides plant.

In another preferred embodiment of the present invention, wherein said sophoridine and analogues are substituted by one or more substitutes independently selected from halogen, C₁-C₆ alkyl, alkenyl, cycloalkyl, substituted alkyl, and alkoxyl, aryl, acyl, acyloxy, hydroxyl, sulphonyl or sulfhydryl., carboxyl or carbonyl.

According to a more preferred embodiment of the present invention, wherein said mammals arc human and livestock selected from pig, cattle, horse, and sheep, and pets selected from dog, cat, mouse, rat, pig, guinea pig and rabbit.

According to another preferred embodiment of the present invention, wherein said pain is caused by advanced tumors, mechanical or chemical trauma, burn, cramp, nervous lesion, infectious or non-infectious inflammatory injury, irritation of foreign body, metabolic disorders or dysendocrinism, neuropathy, and central or peripheral vascular disease.

In general with respect to the pharmaceutical composition as defined above, the active ingredient also includes one or more natural or synthesized or recombinant produced other active ingredients having similar or synergistic effect. These active ingredients include, but not limited to other conventional analgesics, anticancer agents, anti-microorganisms, anti-inflammatory agents and immuno-modulating agents.

According to another preferred embodiment of the present invention, wherein said pharmaceutical compositions are administrated by parenteral or non-parenteral routs that include, but not limited to local, nasal, bronchial, subcutaneous, percutaneous, transmucosal, intravenous, intramuscular, intra-caveties, intra-tumoral routs.

It is another object of the present invention, to provide a use of sophoridine and analogous or derivatives thereof in preparing the medicaments for pain-alleviatives, anti-tumor, anti-microbe, anti-inflammatory and anti-neurodegenerative.

It is still further object of the present invention, to provide a method for treating or alleviating pain comprising administering a pharmaceutical composition described above which containing a therapeutieally effective amount of diquinolizidine-like compound, and one or more pharmaceutically acceptable carriers or excipients.

In a preferred embodiment, wherein said diquinolizidine-like compound comprises, but not limited to, matrine, aloperine, malanine, sophocarpine, oxymatrine, sophoramine and sophoridine, and pharmaceutically acceptable salts thereof.

In a more preferred embodiment, wherein said diquinolizidine-like compound is sophoridine or pharmacentically acceptable salts and derivatives thereof.

### DETAILED DESCRIPTION OF THE INVENTION

The present invention relates to use of natural or chemically synthetic sophoridine and its analogues or derivatives as an analgesic. The present invention further related to pharmaceutical compositions comprising alkaloid compounds having diquinolizidine-like structure selected from the group consisting of sophoridine, sophocarpine, matrine, oxymatrine, malanine, aloperine, sophoramine, and pharmaceutically acceptable salts thereof, and one or more pharmaceutically acceptable carriers or excipients. The pharmaceutical compositions of the present invention can be used for eliminating or alleviating acute or chronic pain, but does not suffering from the undesirable tolerance and addiction with continued usage.

Sophoridine is an alkaloid extracted and purified by Oreknov (A. Oreknov, C.A.,27,4234) from the leguminous plant Sophora alopecuroides. Xuemei Li *et al*. (Xuemei Li *et al*., Chinese Journal of Pharmacology 8(2):153-158,1987 and China Patent No. 93100881.6) demonstrated that sophoridine isolated from the leguminous plant Sophora alopecuroides exhibited tumor cell-killing and tumor cell growth-inhibiting activities based on structural similarity between sophoridine and matrine having inhibitory activity on growth of implanted tumor within a effective dosage range. As well known that both sophoridine and sophocarpine are stereoisomers of matrine isolated the same natural source. Also, Li *et al*. found that this compound does not inhibit the functions of hematopoietic and immunological systems (see Xuemei Li *et al*., *supra*). The researchers further found in clinical trials that sophoridine exhibited a inhibitory activity on growth of choriocarcinoma, chorioadenoma, malignant lymphoma, gastric cancer, and small cell undifferentiated carcinoma of lung to different degrees.

Based upon published research results as described above, the present inventor observed the effects of sophoridinc on more than 200 patient victims suffered from different type of advanced cancers in his clinical trials, and surprisingly found that sophoridine and other diquinolizidine-like alkaloids such as sophocarpine isolated from the leguminous plant Sophora alopecuroides, not only inhibit proliferation of tumor cells but also significantly inhibit or alleviate mild to severe pain caused by malignant growth of cancer tissues. Furthermore, the results of clinical trials show that administration of sophoridine and analogous or derivatives thereof to patients suffering from advanced cancers at higher doses for a long term did not develop any side effects of tolerance and addiction, namely without any of withdrawal symptoms in observed victims.

The present invention further relates to methods and compositions for treating various forms of pain in mammals including human. Although the present invention is expected to be useful for virtually all pain types, it is most potent for such as pain caused by advanced tumors, inflammatory pain, and neuropathic pain. At higher doses, the present invention is also effective in acute pain states such as that induced by mechanical or chemical trauma, bum, cramp, nervous lesion, and infectious or non-infectious inflammatory injury, irritation of foreign body, metabolic disorders or dysendocrinism, neuropathy and central or peripheral vascular disease.

Wherein, inflammatory pain can occur when tissue is damaged, as can result from surgery or due to an adverse physical, chemical or thermal event or to infection by a biologic agent. Although inflammatory pain is generally reversible and subsides when the injured tissue has been repaired or the pain inducing stimulus removed, present methods for treating inflammatory pain have many drawbacks and deficiencies ( for example, shorter drug efficacy durations, drug resistance, antibody development and/or drug dependence and addiction ). In addition, chemically induced pain may occur when a patient is exposed to chemical agents that trigger pain response. Commonly, chemical pain is used to test anesthetic or analgesic efficacy of treatment methods. Furthermore, some examples of neuropathic pain are diabetic neuropathy, pain associated with AIDS infection and treatment, pain due to cancer treatment, , traumatic injury, complex regional pain syndrome and pain due to central or peripheral vascular disease.

And wherein said mammals are human and livestock selected from pig, cattle, horse, and sheep, and pets selected from dog, cat, mouse, rat, pig, guinea pig and rabbit. In a particular preferred embodiment of the present invention, wherein mammals are human.

Using well known conventional techniques, the ordinary skills in the art easy to extract and isolate sophoridine and other alkaloids from leguminous Sophora alopecuroides plant by soaked with a solution of diluted acid, cation and anion exchange chromatography, extracted with organic solvents and recrystallization ( see, for example, Yunguang Wu *et al*., Studies on Sophora alopecuroides, Suppl., 1974; Boguang Zhao *et al*., Chinese Journal of Pharmacology 15(3):182,1980 ). Furthermore, one can prepare salts of sophoridinc having improved solubility or storage stability. These preferred acid addition salts are chlorides, bromides, sulfates, nitrates, phosphates, sulfonates, formates, tartrates, maleates, malates, citratcs, benzoates, salicylates, ascorbates, and the like.

The pharmaceutically acceptable salts of the sophoridine derivatives include the conventional non-toxic salts of the sophoridine derivatives formed, for example, from non-toxic inorganic or organic acids. Such conventional non-toxic acid addition salts include those derived from inorganic acids such as hydrochlorie, hydrobromic, sulfurie, sulfamic, phosphoric, nitric perchloric, and the like; and the acid addition salts prepared from organic acids such as acetic, propionic, butyric, adipic, butanedioic, succinic, glycolic, stearic, lactic, malic, tartaric, citric, ascorbic, maleic, nicotinic, hydroxymaleic, phenylacetic, glutamic, benzoic, salicylic, sulfanilic, fumaric, toluenesulfonic, methanesulfonic, oxalic, and the like.

The pharmaceutically acceptable salts of the present invention are synthesized from the sophoridine derivatives by conventional chemical methods. Generally, such salts are prepared by reacting the free base forms of these compounds with a stoichiometrie amount of the appropriate acid in water or in an organic solvent, or in a mixture of the two; generally, nonaqueous media like ether, ethyl acetate, ethanol, isopropanol, or acetonitrile are preferred.

The sophoridine derivatives are prepared in a number of ways well known to one skilled in the art of organic synthesis. The sophoridine derivatives are synthesized using the methods described elsewhere, together with synthetic methods known in the art of synthetic organic chemistry, or variations thereon as appreciated by those skilled in the art. In addition to salts, the skilled persons in the art of organic synthesis can also prepare other sophoridine derivatives which suitable for various clinical uses and research purposes by well known process such as dehydrogenation, cyclization, dehydrocyclization, oxidation, alkylation, acylation, esterfication, amidation, and the like. Alternatively, skilled persons in the art can also take advantage of known organic synthesis technique to synthesize sophoridine and its analogues or derivatives which have increased anti-tumor and pain-killer activities, and decreased toxic and side-effect.

As used herein, the term "sophoridine derivative" refers to natural occurred or synthetic or semi-synthetic sophoridine or analogue and derivative thereof obtained by substituting with one or more substituents in any position of homocyclic or heterocyclic ring structure of sophoridine molecular. Wherein, said substitutes independently selected from halogen, C₁-C₆ alkyl, alkenyl, cycloalkyl, substituted alkyl, and alkoxyl, aryl, acyl, acyloxy, hydroxyl, sulphonyl or sulfhydryl., carboxyl or carbonyl.

As used herein, the term "sophoridine analogue" refers to compounds having diquinolizidine-like structure either extracted from natural plant sources or chemically synthesized and comprises, but not limited to, matrine, aloperine, malanine, sophocarpine, oxymatrine, sophoramine and sophoridine, and pharmaceutically acceptable salts thereof.

It is worthy of attention that these sophoridine derivatives and analogues described as above are only examples and that other alkaloid compounds which have diquinolizidine-like structure, whether natural occurring or semi-synthetic or synthetic, can also be contemplated by the person skilled in the art without departing from the scope of the invention, and can further be used to prepare pharmaceutical compositions that useful for treatment of pain and/or tumor and/or inflammation.

As an essential ingredient, sophoridine or derivatives or analogues thereof have a excellent analgesic effect, particular used for the patients suffering from advanced tumor. Further, these compounds are useful for suppression, alleviation and treatment of some inflammatory reactive diseases, such as rheumatoid arthritis, osteoarthritis and gouty arthritis.

While not intended to be bound by theory, it is believed that the activity of alkaloids as above may be attributed to the fact that these compounds exert their efforts by increasing concentrations of the neuro-transmitter, acetylcholine. Therefore, it is possible that these compounds are also useful for treating patients suffering from neurodegenerative diseases, senile dementia of the Alzheimer's type (SDAT) and Pakinson's disease (PD).

Alkaloid compounds and pharmaceutically acceptable salts thereof can be administered as they are, or in the form of various pharmaceutical compositions, according to the pharmacological activity and the purpose of administration. The pharmaceutical compositions in accordance with the present invention can be prepared by uniformly mixing an elective amount of compounds or a pharmaceutically acceptable salt thereof, as an active ingredient, with a pharmaceutically acceptable carrier. Such compositions can be prepared for use in parenteral administration, particularly in the form of liquid solutions or suspensions; or oral administration, particularly in the form of tablets or capsules, particularly in the form of powders, emulsions, granulas, nasal drops, or aerosols; or dermal, via, for example, trans-dennal patches.

The composition of the present invention can be conveniently administered in unit dosage form suitable for oral or non-oral administration and may be prepared by any of the methods well known in the field of pharmaceutical industry, for example, as described in Remington's Pharmaceutical Sciences (Mack Pub. Co., Easton, Pa., 1980). Formulations for parenteral administration may contain as common excipients sterile water or saline, polyalkylene glycols such as polyethylene glycol, oils and vegetable origin, hydrogenated naphthalenes and the like. In particular, biocompatible, biodegradable lactide polymer, lactide/glycolide copolymer, or polyoxyethylene-polyoxypropylene copolymers may be useful cxcipients to control the release of the active compounds. Other potentially useful delivery systems for these active compounds include ethylene-vinyl acetate copolymer particles, osmotic pumps, implantable infusion systems, and liposomes. Formulations for inhalation administration contain as excipients, for example, lactose, or may be aqucous solutions containing, for example, glycocholate and deoxycholate, or oily solutions for administration in the form of nasal drops, or as a gel to be applied intranasally. Formulations for parenteral administration may also include glycocholate for buccal administration, a salicylate for rectal administration, or citric acid for vaginal administration. Formulations for trans-dermal patches are preferably lipophilic emulsions.

The alkaloid compounds described as above can be employed as the sole active agent in a pharmaceutical composition. Alternatively, they can be used in combination with other active ingredients including natural or synthesized or recombinant produced other active ingredients having similar or synergistic effect. For instance, these active ingredients include, but not limited to other analgesics or antipyretic analgesics, for example, non-steroidal anti-inflammatory drugs ( NSAIDS_{S}) such as aspirin, phenylbutazone, butyrate derivatives, phenacaine and the like, and narcotic analgesics; anti-cancer agents such as nitrogen mustard, cyclophosphamide, vincristine, taxol, daunomycin and the like; antimicrobial agents such as sorbistat, stamicin, baicalin and the like; and immunological modulator such as interleukin, interferon, thymulin and bulk glycosides of Tripterygium wifordii and the like.

These alkaloid compounds, in particularly sophoridine and derivatives, and pharmaceutically acceptable salts thereof can be administered orally or non-orally, e.g., as an a injection. The concentrations of the alkaloid compounds in a therapeutic composition can vary. The concentration will depend upon factors such as the total dosage of the drug to be administered, the chemical characteristics of the compounds employed, the route of administration, the age, body weight and symptoms of a patient, etc. The pharmaceutical compositions of this invention typically are provided in an aqueous physiological buffer solution containing about 0.1 to 10% w/v compound for parenteral administration. Generally, the therapeutically effective amount of compositions of this invention ranges from about 0.1 to 100mg/kg/day, preferably about 1 to 80mg/kg/day, more preferably about 5 to 50mg/kg/day. In General, a therapeutic amount between 0.05 to 100mg/kg/day, preferably between 0.1 to 80mg/kg/day, and more preferably between 0.5-50mg/kg/day is especially effective for intraperitoneal or intramuscular administering, while 0.01 to 100mg/kg/day, preferably 0.05 to 80mg/kg/day, more preferably 0.1 to 50mg/kg/day for intravenous administering. The skilled artisan will appreciate the exact dosage required to effectively treat a subject and it should be individually determined depending upon various factors including the dosage form employed and the route of administration utilized, the severity of the disease to be treated, previous treatments, the general health, age and weight of the subject, and the sensitivity, tolerance of patients toward the treatments by clinicians.

Tablets can be prepared using excipients such as lactose, glucose, sucrose, mannitol and methyl cellulose, disintegrating agents such as starch, sodium alginate, calcium carboxymethyl cellulose and crystalline cellulose, lubricants such as magnesium stearate and talc, binders such as gelatin, polyvinyl alcohol, polyvinyl pyrrolidone, hydroxypropyl cellulose and methyl cellulose, surfactants such as sucrose fatty acid ester and sorbitol fatty acid ester, and the like in a conventional manner.

Granules can be prepared using excipients such as lactose and sucrose, disintegrating agents such as starch, binders such as gelatin, and the like in a conventional manner. Powders can be prepared using excipients such as lactose and mannitol, and the like in a conventional manner. Capsules can be prepared using gelatin, water, sucrose, gum arabic, sorbitol, glycerin, crystalline cellulose, magnesium stearate, tale, and the like in a conventional manner.

Syrup preparations can be prepared using sugars such as sucrose, water, ethanol, and the like in a conventional manner.

Injectable preparations can be prepared using solvents such as water, physiological saline, vegetable oils (e.g., olive oil and peanut oil), ethyl oleate and propylene glycol, solubilizing agents such as sodium benzoate, sodium salicylate and urethane, isotonicity agents such as sodium chloride and glucose, antibiotics such as pcnicillin and streptomycin and other anti-fungai agents, preservatives such as phenol, cresol, p-hydroxybenzoic ester and chlorobutanol, antioxidants such as ascorbic acid and sodium pyrosulfite, and the like in a conventional manner.

It will be understood that the administration forms mentioned above arc only examples, and that other modes of administration may also be contemplated by the person skilled in the art without departing from the scope of the invention.

Based on the surprising discovery of the present inventors described as above, and have demonstrated inhibitory activity of sophoridine and analogues thereof on tumors such as choriocacinoma, chorioadenoma, malignant lymphoma, gastric cancer, and small cell undifferentiated carcinoma of lung (see Xuemei Li *et al*., Chinese Journal of Pharmacology 8(2):153-158,1987 and China Patent No.93100881.6 ), it is possible to prepare the pharmaceutical compositions having both analgesic and anti-cancer activities using sophoridine or derivatives thereof as an active ingredient. In addition, it is also possible to combing sophoridine or derivatives thereof as an auxiliary ingredient with other known analgesic and /or anti-tumor agents, in order to improve effects of these therapeutic medicaments and significant reduce administering doses of certain conventional analgesics such as narcotic analgesics which trend to the adverse effects of tolerance and addiction with repcated use, or certain chemical anti-tumor agents such as alkylating agents and antimetabolites which trend to hematopoietic disorders of bone marrow and depression of immunological functions, thereby decrease the sufferings of patients due to take these medicaments for long period of time and significantly improve the living standard of survivals. Similarly, based on the demonstrated analgesic effect of sophoridine and analogues and derivatives thereof and the discovered synergistic effect between sophoridine and opioid analgesics ( see example 3 ), it is also possible to combination sophoridine or derivatives thereof as an auxiliary ingredient with opioid analgesics or NSAIDS_{S} or anti-gout drugs, to obtain a new pharmaceutical composition useful for suppression, alleviation and treatment of pain from various seasons, and some inflammatory diseases, such as rheumatoid arthritis, osteoarthritis and gouty arthritis, to improve therapeutic effects of these drugs and effectively decrease administering doses. Furthermore, our preliminary study found that a possible mechanism of analgesic effect of sophoridine or derivatives is significant increasing acetylcholine level in central nervous system (CNS). Therefore, sophoridine and derivatives thereof arc also useful for treating patients suffering from neurodegenerative diseases, senile dementia of the Alzheimer's type (SDAT) and Pakinson's disease (PD).

Further details of the present, invention will be apparent from the following Examples which are included by way of illustration, not by way of limitation, of this invention. This application is intended to cover those changes and substitutions which may be made by those skilled in the art without departing from the spirit and the scope of the appended claims. All publications, patents, and patent applications cited herein are hereby incorporated by reference in their entirety.

### EXAMPLES

### Example 1 : Analgesic effect of sophoridine on mice model ( hot plate method )

The Analgesic activity of sophoridine was tested in vivo in mice experimental models, using Hot Plate Method which are known to evaluate the pain in animals.

The method that was used is described by Eddy N. B. and Leimbach D. (J. Pharm. Exp. Ther. 107: 385-393, 1953) and somewhat modification is made in the present invention.

Male Kumming mice weighing 18-22 g were used as experimental model. The analgesic effect of the sophoridine was evaluated by analysing the behavioral response of the animals on a hot surface at 55°C±0.5°C. The alkaloid compounds being tested were administered intravenouslly through the tail vein 10 minutes before starting the test.

Briefly, the process consisted of placing the animals on the plate and keeping them in a plastic cylinder 22 cm diameter and 11 cm high. The time the animals took to lick a paw or jump off the hot surface was determined. The animals were selected before starting the test that those response latency ( the pain threshold values before administrations ) longer than 30 seconds were excluded from the experiment The certificated animals divided into six groups ( 10 animals/group ). Five experimental groups received 25, 20, 15, 10 and 5 mg/kg of the sophoridine suspended in a normal saline solution, respectively. The control group received treatment only with the solvent (normal saline).

10 minutes after administering the alkaloids being tested, the test was repeated and the maximum response latency time (seconds) took the animals to lick the hindlimb or jump off and run away was again recorded. Those animals that did not jump off after 60 seconds were removed from the plate to avoid any injuries. The results are expressed as the pain threshold values (seconds ) before and 10 minutes after administrations, and shows as following Table 1.

**Table 1 :**

| Analgesic effect of sophoridine on mice model ( hot plate method ) | | | |
|---|---|---|---|
| Groups (n=10) | Administering doses (mg/kg) | Pain thresholds (seconds) ( X±SD) | |
| | | Before administration | After administration |
| Norm. saline | ― | 12.8±3.91 | 15.3±5.77 |
| Sophoridine | 25 | 16.4±6.48 | 48.6±19.14*** |
| | 20 | 12.4±4.25 | 57.1±6.10*** |
| | 15 | 14.7± 4.62 | 38.5 ±17.19** |
| | 10 | 11.7±4.59 | 24.8 ±15.99 |
| | 5 | 16.3±6.02 | 25.8±20.74 |
| Statistically significant difference from control saline alone group (the baseline): **p<0.07; ***p<0.001. | | | |

It can be seen from the data as shown in table 1 that, after administering the alkaloid sophoridine, the pain thresholds of the treated animals are increased in dose-dependent mode. These results indicated that the alkaloid derived from Sophora alopecuroides, sophoridinc exhibited a good analgesic activity in mice models, and may useful for inhibit or alleviate pain caused by various seasons in mammals as an analgesic.

### Example 2 : Analgesic effect of sophoridine on mice models (writhing method)

The Analgesic activity of sophoridine was also tested in vivo in mice experimental models, using acetic acid induced writhing Method which are known to evaluate the pain in animals. Writhing lest is a standard procedure for detecting and comparing analgesic activity in laboratory animals, and generally correlates well with human efficacy.

30 Male and 30 female Kunming mice weighing 18-22 g were used as experimental models. The mice received intraperitoneal injection of 0.2 ml/animal of 0.6% acetic acid causes them to exhibit the abdominal stretching movements, or writhe. Then, the animals randomly divided into six groups, each group includes 10 animals. Five experimental groups intravenouslly received 25, 20, 15, 10 and 5 mg/Kg of the sophoridine suspended in a normal saline solution via the tail vein, respectively.

The frequency of writhing syndrome was counted for 5 minutes from 15 minutes after the injection of spophoridinc or placebo. The control group received treatment only with the solvent (normal saline ). The results were showed as following Table 2.

**Table 2:**

| Analgesic effect of sophoridine on mice models ( writhing method ) | | |
|---|---|---|
| Groups (n=10) | Administering doses (mg/kg) | Numbers of writhe (X±SD) |
| Norm. saline | ― | 33.1±7.40 |
| Sophoridine | 25 | 0*** |
| | 20 | 1.5 ±1.35*** |
| | 15 | 9.9±6.3*** |
| | 10 | 14.1±9.64*** |
| | 5 | 23.2±6.43** |
| Statistically significant difference from control saline alone group (the baseline): **p<0.01; ***p<0.001. | | |

It can be seen from the data as shown in table 1 that, after administering the alkaloid sophoridine, the numbers of nociceptive response (licking) of the treated animals were decreased in a clearly dose-dependent mode. These results indicated that sophoridine, the alkaloid derived from Sophora alopecuroides, exhibited a good analgesic activity in mice models, and may useful for inhibit or alleviate pain caused by various seasons in mammals as an analgesic.

### Example 3 : A comparison of analgesic effects between sophoridine and morphine, and synergistic effect of the two medicaments

A comparative test of analgesic effects between sophoridine and morphine was performed and the possible synergistic effect of the two drugs further was observed by means of Hot Plate Method.

Screening the experimental animals and grouping according to Example 1. In five experimental groups, animals of each group received 25, 20, 15, 10 and 5 mg/kg of the sophoridine suspended in a normal saline solution, respectively. Animals of two positive controls were orally administrated morphine hydrochloride ( Shenyang first pharmaceutical factory, Shenyang, China) and sophoridine + morphine in indicated doses in Table 3, respectively. At 15, 30, 60 and 90 minutes after administration, the pain thresholds (response latency time, seconds) of each animal was recorded. The results were summarized in Table 3 below.

**Table 3 :**

| A comparison of analgesic effects and synergistic activity between sophoridine and morphine | | | | | | |
|---|---|---|---|---|---|---|
| Groups (n=10) | Admin. doses (mg/kg) | Pain thresholds before admin. | Pain thresholds after admin. (seconds) (X±SD) | | | |
| | | | 15 min | 30 min | 60 min | 90 min |
| Norm. saline Sophoridine | ― | 17.4±4.9 | 16.2±5.5 | 17.7±8.1 | 18.9 ±6. 1 | 18.2 ±6. 1 |
| | 25 | 15.4±4.0 0 | 44.3±14.9*** | 28.3±11.4** | 20.3±4.9* | 18.8±6.3 |
| | 20 | 16.9±2.5 | 36.8±18.2** | 24.6±7.7** | 23.0±9.9 | 19.3±6.6 |
| | 15 | 16.45±3.9 | 32.9±19.2* | 31.3±17.5* | 15.2±10.2 | 15.8±5.7 |
| | 10 | 18.6±4.9 | 20.2±7.2 | 28.0±18.3 | 12.6±5.9 | 20.5±15.0 |
| | 5 | 17.45±2.6 | 17.5±3.3 | 16.6±6.5 | 16.0±4.5 | 19.3±5.4 |
| Morphine Sophoridine | 10 | 19.7±4.4 | 35.1±15.5** | 44.8±14.6*** | 32.4±16.2* | 27.5±16.5 |
| +Morphine | 20+10 | 19.1±4.4 | 60±0*** | 57.2±8.9*** | 19.6±12.7*** | 39.9±17.0** |
| Statistically significant difference from blank control group: *p<0.05; **p<0.01; ***p<0.001. | | | | | | |

It can be further seen that from the data as shown in table 1, the experimental animals exhibit a longer pain latency 15 minutes after administering sophoridine in higher doses comparing to the saline control group. Therefore, it is can be concluded that sophoridine in higher doses have a comparable or better analgesic activity for the animals comparing to the conventional analgesic, morphine hydrochloride. Also, It has been surprisingly found that in the treated mice, administering of the two drugs in combination provided a synergistic analgesic effect compared with administration of either drug alone, and so that a lower dosages of the conventional analgesic agent such as morphine may be used.

### Example 4 : Effect of the opioid receptor antagonist naloxone on morphine- and sophoridine-induced analgesia

This example describes experiments in which the effect of the morphine antagonist, naloxone, ( Beijing fourth pharmaceutical factory, Beijing, China ) upon morphine- and sophoridine-induced analgesia in the mouse writhing test described above.

Following procedures of the previous Examples, screening and grouping of experimental animals was carried out. The animals of five experimental groups were intravenously injected with (1) morphine antagonist, naloxone alone (2mg/kg); (2) sophoridine alone (20mg/kg ); (3) morphine hydrocbloride alone ( 10mg ); (4) naloxone ( 2mg/kg ) plus morphine hydrochloride ( 10mg ); and (5) naloxone ( 2mg/kg ) plus sophoridine (20mg/kg ), respectively. The animals of negative control were injected with normal saline. The pain thresholds (response latency time, seconds) of each animal was recorded at 15, 30, 60, and 90 minutes after administrations, respectively. The results are summarized in Table 3 below.

**Table 4 :**

| Effect of the opioid receptor antagonist naloxone on morphine- and sophoridine- induced analgesia | | | | | | |
|---|---|---|---|---|---|---|
| Groups ( n=10) | Admin. doses (mg/kg) | Pain thresholds before admin. (seconds) (X±SD) | Pain thresholds after admin. (seconds) (X±SD) | | | |
| | | | 15 min | 30 min | 60 min | 90 min |
| Norm. saline | ― | 16.5±3.5 | 18.2±6.5 | 19.7±6.0 | 16.9±6.5 | 17.5±6.0 |
| Naloxone | 2 | 18.5±2.5 | 17.4±7.7 | 15.8±6.0 | 19.0±8.7 | 18.1±4.0 |
| Sophoridine | 20 | 17.9±2.6 | 34.5±16.2** | 23.8±7.0 | 21.8±8.9 | 18.3±6.6 |
| Morphine Naloxone⁺ | 10 | 19.8±4.0 | 36.0±13.5 | 43.5±12.5*** | 31.6±15.8* | 26.3±15.5 |
| Morphine Naloxone⁺ | 2+1 | 15.7±3.6 | 17.9±15.6 | 26.1±16.5 | 24.7±15.3 | 16.0±17.0 |
| Sophoridine | 2+20 | 18.3±5.1 | 33.2±10.0** | 21. 9±13.4 | 17.7±6.0 | 16.0±5.7 |
| Statistically significant difference from control saline group: *p<0.05, **p<0.01. | | | | | | |

Table 4 shows a comparison of the latency of the pain response in the mice hot plate test described above. As shown in this table, while naloxone ( 2.0 mg/kg ) was effective in counteracting the analgesic effect of morphine (10 mg/kg ), it was ineffective in counteracting the effect of sophoridine (20 mg/kg ) on analgesia (as evidenced by latency time (seconds) of nociceptive response ), indicating that morphine and sophoridine act by different mechanisms in inducing analgesic. In other word, these results clearly indicated that analgesic activity of sophoridine does not through activating opioid receptor in CNS.

### Example 5: Effect of the total alkaloids derived from leguminous Sophora alopecuroides plant on mice model

This example describes experiments in which the effect of the total alkaloids including matrine, aloperine, malanine, sophocarpine, oxymatrine, sophoraminc and sophoridine derived from leguminous Sophora alopecuroides plant upon mice model in the hot plate test as described in Example 1. The results were showed in Table 4 below.

**Table 5 :**

| Effect of the total alkaloids derived from leguminous Sophora alopecuroides plant on mice model | | | |
|---|---|---|---|
| Groups (n=10) | Administering doses (mg/kg) | Pain thresholds (seconds) (X±SD) | |
| | | Before administration | After administration |
| Norm. saline Total alkaloids | ― | 10.6±2.56 | 14.3±3.65 |
| | 25 | 15.3±6.20 | 45.5±16.10**** |
| | 20 | 11.6±5.25 | 50.8±10.17*** |
| | 15 | 13.8±6.50 | 35.6±12.15** |
| | 10 | 12.3±3.85 | 19.2±10.80 |
| | 5 | 15.3±5.80 | 23.7±15.75 |
| Statistically significant difference from control saline group: **p<0.07 ; ***p<0.001. | | | |

It can be seen from the data as shown in table 1 that, after administering the total alkaloids ( 15-25mg/kg ) derived from leguminous Sophora alopecuroides plant, the pain thresholds of the treated animals are increased in dose-dependent mode. Just like sophoridine, these results indicated that the alkaloids including matrine, aloperine, malanine,sophocarpine, oxymatrine, sophoramine and sophoridine derived from Sophora alopecuroides plant exhibited a good analgesic activity in mice models, and may also useful for inhibit or alleviate pain caused by various seasons in mammals as an analgesic.

## Claims

1. A pharmaceutical compositions used for treatment or alleviation of pain caused by various reasons in mammals, comprising a therapeutically effective amount of sophoridine or analogues or derivatives thereof as a essentially active ingredient, and one or more pharmaceutically acceptable carriers or excipients.

2. A pharmaceutical compositions according to claim 1, wherein said sophoridine and analogues or derivatives thereof are substituted by one or more substitutes independently selected from halogen, C₁-C₆ alkyl, alkenyl, cycloalkyl, substituted alkyl, and alkoxyl, aryl, acyl, acyloxy, hydroxyl, sulphonyl or sulfhydryl., carboxyl or carbonyl

3. A pharmaceutical compositions according to claim 1, wherein said sophoridinc and analogues or derivatives thereof are selected from the group consisting of Matrine, Aloperine, Malanine, Sophocarpine, Oxymatrine, Sophoramine and Sophoridine, and pharmaceutically acceptable salts thereof.

4. A pharmaceutical compositions according to claim 1, wherein said mammals are human and livestock selected from pig, cattle, horse, and sheep, and pets selected from dog, cat, mouse, rat, pig, guinea pig and rabbit.

5. A pharmaceutical compositions according to claim 1, wherein said mammals are human.

6. A pharmaceutical compositions according to claim 1, wherein said pain is caused by advanced tumor, mechanical or chemical trauma, burn, cramp, nervous lesion, infectious or non-infectious inflammatory injury. irritation of foreign body, metabolic disorders or dysendocrinism, neuropathy and central or peripheral vascular disease.

7. A pharmaceutical composition according to claim 1, wherein said sophoridine and analogues or derivatives thereof are obtained from natural or chemical synthetic sources.

8. A pharmaceutical composition according to claim 1, wherein said pharmaceutical composition also exhibits antineoplastic, antimicrobial, anti-inflammatory, and anti-neurodegenerative activities.

9. A pharmaceutical composition according to claim 1, wherein the pharmaceutical composition further contains one or more natural or synthesized other active ingredients having similar or synergistic effect.

10. A pharmaceutical composition according to claim 9, wherein said other active ingredients are selected from the group consisting of conventional analgesics, antineoplastic agents, anti-microbial agents, anti-inflammatory agents and immuno-modulating agents.

11. A pharmaceutical composition according to claim 1, wherein said composition is administrated via local, nasal, bronchial, subcutaneous, percutaneous, transmucosal, intravenous, intramuscular, intra-caveties, intra-tumoral.

12. A pharmaceutical composition according to claim 1, wherein said therapeutically effective amount of sophoridine or analogues or derivatives thereof in the composition is about 0.01 to 100mg/kg body weight.

13. A pharmaceutical composition according to any one of claims 1-11, wherein the sophoridine and analogues or derivatives thereof used in producing the medicaments for preventing or tresting pain, tumor, gout, inflammatory and neurodegenerative diseases.
